# EUROPEAN PATENT SPECIFICATION

(11) **EP 1 056 850 B1**
(45) Date of publication and mention of the grant of the patent: **18.08.2004**
(21) Application number: 99906341.5
(22) Date of filing: 23.02.1999
(51) Int. Cl.: C12N 15/12, C12N 15/62, C12N 15/85, C12N 15/86

(54) **BIOTIN-BINDING RECEPTOR MOLECULES**
BIOTINBINDENDE REZEPTORMOLEKÜLE
MOLECULES RECEPTRICES LIANT LA BIOTINE

(30) Priority: 23.02.1998 GB 9803757; 24.06.1998 GB 9813653
(43) Date of publication of application: 06.12.2000
(73) Proprietor: Ark Therapeutics Limited, London W1T 6DE (GB)
(72) Inventor: YLA-HERTTUALA, Seppo A.I. Virtanen Institute, FIN-70211 Kuopio (FI); KULOMAA, Markku, FIN-Jyvaskyla 40351 (FI); LEHTOLAINEN, Pauliina A.I. Virtanen Institute, FIN-70211 Kuopio (FI); MARJOMAKI, Varpu, FIN-40100 Jyväskylä (FI); AIRENNE, Kari, FIN-Jyvaskyla 40351 (FI)
(74) Representative: Perry, Robert Edward
(86) International application number: PCT/GB1999/000546
(87) International publication number: WO 1999/042577

(56) References cited:
- EP-A- 0 359 347
- WO-A-87/05026
- WO-A-97/19957
- KULOMAA M S (REPRINT) ET AL: "PRODUCTION OF RECOMBINANT AVIDIN AND AVIDIN - FUSION PROTEINS IN BACTERIAL AND INSECT CELLS" FASEB JOURNAL, (24 APR 1995) VOL. 9, NO. 6, PP. A1395. ISSN: 0892-6638., XP002112828
- MARJOMAKI V (REPRINT) ET AL: "Efficient transport of avidin fusion proteins into late and early endosomes." MOLECULAR BIOLOGY OF THE CELL, (DEC 1996) VOL. 7, SUPP. [S], PP. 2631-2631. PUBLISHER: AMER SOC CELL BIOL, PUBL OFFICE 9650 ROCKVILLE PIKE, BETHESDA, MD 20814. ISSN: 1059-1524., XP002112829
- RIHOVA B: "Targeting of drugs to cell surface receptors." CRITICAL REVIEWS IN BIOTECHNOLOGY, (1997) 17 (2) 149-69. REF: 147 , XP002112830

## Description

### Field of the Invention

This invention relates to membrane-spanning proteins having biotin-binding activity.

### Background to the Invention

Biotin (vitamin H) is a readily water-soluble substance found at low concentrations in blood and tissues. The biological role of biotin is as a carrier of activated CO₂ and it permits the transfer of CO₂ to acceptors without the need for additional free energy. The activated carboxybiotin is usually attached to an enzyme that is required for the formation of carboxybiotin. For example, biotin may be attached to pyruvate carboxylase which, in the presence of acetyl CoA, catalyses the formation of carboxybiotin and the subsequent transfer of the activated carboxyl group to pyruvate, to form oxaloacetate.

Biotin also binds with one of the highest naturally known affinities to avidin, a 63 kDa glycoprotein from chicken egg white, and to streptavidin, a non-glycosylated protein from the bacterium *Streptomyces avidinii*. The binding is almost irreversible in character (Ka 10¹⁵ mol⁻¹). The affinity between avidin and biotin has proved very useful in a wide variety of bioanalytical applications. For example, the avidin-biotin complex has been used successfully in a wide variety of detection systems where target molecules are combined with biotin through its carboxy terminus, to form biotinylated molecules which may be easily detected or separated from solution. Biotinylation can occur without changing the biological or physiochemical properties of the various molecules and without affecting the binding capacity of the biotin prosthetic group to avidin.

WO-A-87/05026 discloses the isolation of a DNA sequence encoding streptavidin and a fusion of the streptavidin gene and a gene encoding the human LDL receptor. The fused gene expresses a protein which consists of streptavidin at the N-terminal region of the fused protein and the LDL receptor protein at the C-terminal region of the fused protein. The fused gene may be inserted in an expression vector and used to transform a host cell. The presence of the fusion streptavidin-LDL receptor protein at a cell surface may be determined by addition of blood cells coupled to biotinylated bovine serum albumin.

Kulomaa et al, FASEB J. 9(6) :A1395 (1995), discloses the construction of several avidin fusion protein vectors, including a fusion protein consisting of the avidin protein fused to the chicken progesterone receptor B which has been expressed in *Escherichia coli.*

Marjomaki et al, "Molecular Biology of the Cell" (Dec. 1996), Vol. 7, supp. 5, pp 2631, pub. Am. Chem. Soc. Cell Bio., discloses the use of a Semliki forest virus expression system in order to obtain the transient expression of a chimeric protein containing the transmembrane domain and part of the cytoplasmic domain of the cation-independent mannose 6-phosphate receptor fused to a recombinant avidin in BHK cells.

### Summary of the Invention

According to a first aspect of the present invention, a protein comprises a membrane-spanning domain of an endocytotic receptor and an extracellular domain that comprises biotin-binding activity, for use in therapy.

According to a second aspect of the invention, a nucleic acid for use in therapy encodes a protein as defined above.

According to a further aspect of the present invention, a novel protein comprises a membrane-spanning domain of scavenger receptor class A and an extracellular domain that comprises biotin-binding activity.

Proteins of the present invention may comprise a cytoplasmic domain, a membrane-spanning domain and an extracellular domain, wherein the extracellular domain comprises biotin-binding activity. The extracellular domain may comprise avidin or streptavidin functional activity.

Using proteins or nucleic acid molecules of this invention, it is possible to target biotinylated molecules to specific sites in tissues. Molecules targeted in this way may be taken up by the tissues or cells by endocytosis, allowing the molecules to exert their effects within or on the cell.

### Description of the Drawings

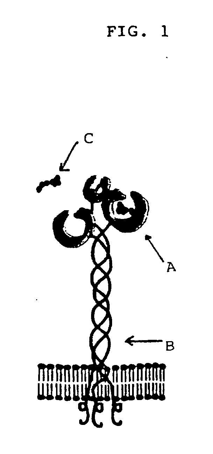

Figure 1 is a schematic illustration of a fusion protein of the present invention, where A represents avidin and B represents the membrane-spanning domain of an endocytotic receptor (and C represents biotin);
Figure 2 is a schematic illustration of a cloning strategy using a shuttle vector; and
Figure 3 is a schematic illustration of a cloning strategy using a retrovirus vector.

### Description of the Invention

Proteins of the present invention may be produced using conventional recombinant DNA technology. Typically, a DNA sequence coding for the functional domain of a biotin-binding protein such as avidin, streptavidin or a related protein, is engineered into a genetic construct which comprises a DNA sequence coding for a protein having membrane-spanning properties. Examples of avidin and streptavidin-related proteins include AVR-1-AVR-5, AVR-X-AVR-V, Stv1 and Stv2.

The individual domains of the fusion protein may be amplified by polymerase chain reaction or isolated from the parent cDNA using restriction enzyme digestion, isolation and purification, e.g. using gel electrophoresis, and subsequent ligation, e.g. using DNA ligase. The fusion protein construct may then be transfected into any suitable host cell, cultured and isolated using standard protein purification techniques.

The construct may also be used as naked DNA or as a plasmid/liposome, plasmid/polyethyleneimine, plasmid/dendrimer or plasmid/peptide complex.

Alternatively, the construct may be introduced into a replication-deficient virus which can be used to target the construct to specific sites in vivo. For example, the construct may be a retroviral vector comprising the appropriate cDNA for the fusion protein. A replication-deficient retrovirus, e.g. Moloney murine retrovirus, may then be used for the stable transfection of target cells and tissues. Other viruses that can be used include replication-deficient adenoviruses, adeno-associated viruses, herpes viruses, papilloma viruses and sinibis viruses. Additional viruses will be apparent to those skilled in the art.

In addition to the functional domains of avidin, streptavidin or related protein, the fusion protein will typically comprise the membrane-spanning domains of endocytotic receptors. The use of these receptors enables the uptake of biotinylated molecules into a target cell. Suitable receptors that may be used in this invention include the scavenger receptor class A, low density lipoprotein receptor, very low density lipoprotein receptor, transferrin receptor and the LOX-1 receptor. The fusion protein may also comprise a linker between the receptor protein and the avidin peptide sequences. The linker may be any length, provided that the functional activity of the different components of the fusion protein is retained.

In general, the fusion between avidin or streptavidin peptide sequences and the receptor peptide sequences is between the extracellular domain of the receptor protein and any site outside of the biotin-binding site of avidin or streptavidin.

The following Example illustrates the invention. Example

A DNA construct was created between the bovine scavenger receptor class A (ScR) (Kodama *et al*. (1990) Nature 343:531-535) and avidin (Green (1975) Adv. Prot. Chem. 29:85-133), which codes for a protein having a ScR cytoplasmic domain, membrane-spanning domain and α-helical coiled domain, ligated to a biotin-binding domain. The complete amino acid sequence of the fusion protein is shown in SEQ ID No 2 where amino acids 1-53 represent the cytoplasmic domain; amino acids 55-79 represent the transmembrane domain; amino acids 81-111 represent a spacer domain; and amino acids 113-272 represent the α-helical coiled domain. Amino acids 273-400 represent the mature avidin peptide sequence derived from avidin cDNA (Gope *et al*. (1987) Nucleic Acid Res. 15:3595-3606) lacking a secretion signal.

Briefly, the cDNA for ScR was obtained from cultured cells previously transfected with a plasmid (PLScRNL) containing the ScR cDNA with an internal Rous Sarcoma Virus promoter and *Hin*dIII restriction sites. The isolated cDNA was then inserted into a *Hin*dIII site of the retrovirus vector pLS1ARNL. The avidin cDNA was produced by the polymerase chain reaction and then inserted into the retrovirus vector at a Sty 1 restriction site on the ScR cDNA. The cDNA embodying the invention is shown as SEQ ID No 1, where nucleotides 1-989 represent a long terminal repeat from Mo-MuSV; nucleotides 1071-2270 represent the coding region for the fusion protein; nucleotides 2376-3101 represent an untranslated region from bovine scavenger receptor I cDNA; nucleotides 3107-3376 represent an RSV promoter region; nucleotides 3727-4522 represent a neo R gene; and nucleotides 4540-5177 represent a long terminal repeat from Mo-MuLV.

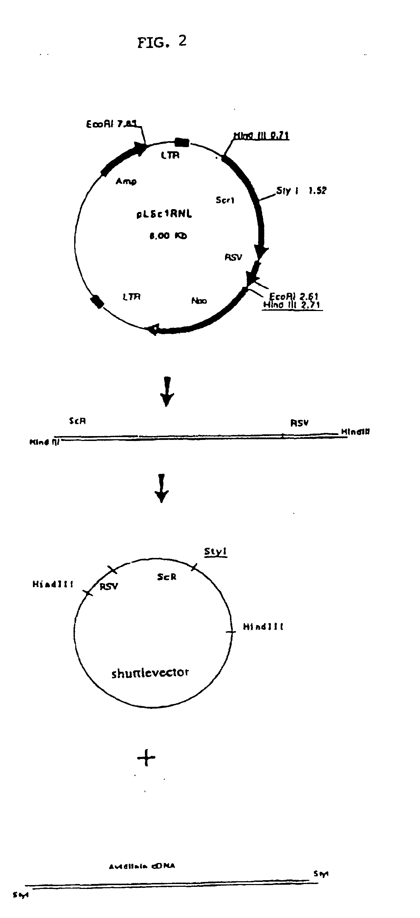

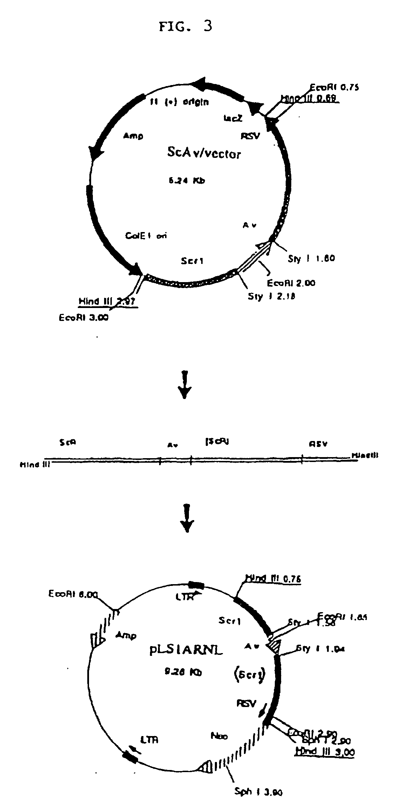

Figs. 2 and 3 refer to processes used in this Example. More specifically, Fig. 2 shows how the ScR cDNA with an internal RSV promoter was cut from plasmid pLScRNL by HindIII and cloned into a HindIII site of a shuttle vector. Fig. 3 shows how the ScR-avidin-RSV cDNA was cloned into a retrovirus vector pLRNL HindIII site.

The expression of the fusion protein in cells transfected with the vector can be confirmed by Northern blotting and immunocytochemical staining with an antibody raised against avidin.

The experiments revealed that the full mRNA transcript was translated into 55 kDa monomers, which were able to form secondary structures of 110 kDa dimers attached by S-S bonds under non-reducing conditions. Approximately 110 kDa dimeric and 55 kDa monomeric peptides were detected, using denaturing conditions. The result is comparable to the computer calculation for the monomeric fusion protein, 45 kDa. In non-denaturing conditions (i.e. using acetylation prior to Western blotting), the strongest signal was approximately 220 kDa which was denatured to an approximately 110kDa dimer and a 55 kDa monomer, suggesting the formation of tetramers. The presence of the 220 kDa protein was also verified using chemical cross-linkers, e.g. NHS-esters. The results show that avidin remains soluble and is capable of forming tetramers even when attached to membrane-spanning domains of endocytotic receptors.

The fusion protein was shown to be a functional protein capable of binding FITC-biotin when analysed by confocal microscopy and atomic force microscopy. Untransduced cells and cells transfected with a retrovirus vector containing the *LacZ* gene were used as controls. No non-specific binding of biotin probes to *LacZ*-transduced control cells was detected by atomic force microscopy. As expected, the transfected cells showed specific binding that was repeatably measurable in unfixed samples. The measured binding forces were multiples of the average 149± 19pN (mean ±sd), which is, as also expected, within the range of the earlier reported biotin-streptavidin binding force of 160 pN (Florin *et al* (1994), Science 264:415-417).

Functionality of the construct can also be confirmed *in vivo* by showing the binding of fluorescently-labelled biotin molecules to cells having the fusion protein construct, using FACS analysis.

The functional activity of the fusion protein in *vivo* was analysed in a rat malignant glioma model. BT4C wild-type glioma cells were implanted intracranially in the right corpus callosum at a depth of 2.5 mm in the brain of inbred BDIX female rats. The growth of tumors was monitored frequently with high resolution MRI (magnetic resonance imaging). Three weeks after tumor cell inoculations, pseudotyped retrovirus carrying cDNA for the fusion protein or LacZ gene in titers of 2 x 10⁶ cfu/ml and 1.3 x 10⁶ cofu/ml, respectively, was transferred into the tumor, firstly at a depth of 2.5 mm and then at a depth of 1.5 mm, with a 10 minute interval. Gene transfer was repeated after two days of growth. Animals were sacrificed and perfusion-fixed with 4% PFA 3 days after the last injection. Brains were removed and divided at the injection site into two coronal pieces, sectioned on ice and analysed with immunoreactivity against anti-avidin antibody. The results showed that the fusion protein was expressed *in vivo* in rat malignant glioma. Protein was detected in glioma cells and in ring-like structures resembling vascular endothelial cells in tumor blood vessels.

## Claims

1. A protein comprising a membrane-spanning domain of an endocytotic receptor and an extracellular domain that comprises biotin-binding activity, for use in therapy.

2. A protein according to claim 1, which further comprises a cytoplasmic domain.

3. A protein according to claim 1 or claim 2, wherein the extracellular domain comprises a biotin-binding domain of avidin or streptavidin.

4. A protein according to any preceding claim, wherein the receptor is scavenger receptor class A.

5. A protein according to claim 1, which comprises SEQ ID No. 2.

6. A nucleic acid molecule encoding a protein according to any of claims 1 to 5, for use in therapy.

7. A protein comprising a membrane-spanning domain of scavenger receptor class A and an extracellular domain that comprises biotin-binding activity.

8. A protein which comprises SEQ ID No. 2.

9. A nucleic acid molecule encoding a protein according to claim 7 or claim 8.

10. A recombinant expression vector comprising a nucleic acid molecule according to claim 9.

## Patentansprüche

1. Protein, umfassend eine membran-überspannende Domäne eines endozytotischen Rezeptors und eine extrazelluläre Domäne, die biotin-bindende Aktivität umfaßt, zur therapeutischen Verwendung.

2. Protein nach Anspruch 1, welches femer eine zytoplasmatische Domäne umfaßt.

3. Protein nach Anspruch 1 oder Anspruch 2, wobei die extrazelluläre Domäne eine biotin-bindende Domäne von Avidin oder Streptavidin umfaßt.

4. Protein nach irgendeinem der vorhergehenden Ansprüche, wobei der Rezeptor ein Abfangrezeptor der Klasse A ist.

5. Protein nach Anspruch 1, welches die SEQ-ID-Nr. 2 umfaßt.

6. Nukleinsäuremolekül, kodierend für ein Protein nach irgendeinem der Ansprüche 1 bis 5, zur therapeutischen Verwendung.

7. Protein, umfassend eine membran-überspannende Domäne eines Abfangrezeptors der Klasse A und eine extrazelluläre Domäne, die biotin-bindende Aktivität umfaßt.

8. Protein, welches die SEQ-ID-Nr. 2 umfaßt.

9. Nukleinsäuremolekül, kodierend für ein Protein nach Anspruch 7 oder Anspruch 8.

10. Rekombinanter Expressionsvektor, umfassend ein Nukleinsäuremolekül nach Anspruch 9.

## Revendications

1. Protéine qui comprend un domaine de récepteur endocyte qui s'apparie à la membrane, et un domaine extracellulaire qui comporte une activité de liaison à la biotine, destinée à être utilisée en thérapie.

2. Protéine selon la revendication 1, qui comprend en outre un domaine cytoplasmique.

3. Protéine selon la revendication 1 ou la revendication 2, dans laquelle le domaine extracellulaire comporte un domaine de liaison à la biotine de l'avidine ou de la streptadivine.

4. Protéine selon l'une quelconque des revendications précédentes, dans laquelle le récepteur est un récepteur de piégeage de classe A.

5. Protéine selon la revendication 1, qui comprend la SEQ ID n° 2.

6. Molécule d'acide nucléique qui code une protéine selon l'une quelconque des revendications 1 à 5, destinée à être utilisée en thérapie.

7. Protéine qui comprend un domaine de récepteur de piégeage de classe A qui s'apparie à la membrane, et un domaine extracellulaire qui comporte une activité de liaison à la biotine.

8. Protéine qui comprend la SEQ ID n° 2.

9. Molécule d'acide nucléique qui code une protéine selon la revendication 7 ou la revendication 8.

10. Vecteur d'expression recombiné qui comporte une molécule d'acide nucléique selon la revendication 9.


### SEQUENCE LISTING

(1) GENERAL INFORMATION:
   (i) APPLICANT:
      (A) NAME: Eurogene Limited
      (B) STREET: Marquis House, 67/68 Jermyn Street
      (C) CITY: London
      (E) COUNTRY: United Kingdom
      (F) POSTAL CODE (ZIP): SW1Y 6NY
   (ii) TITLE OF INVENTION: BIOTIN-BINDING RECEPTOR MOLECULES
   (iii) NUMBER OF SEQUENCES: 2
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version $1.30 (EPO)
(2) INFORMATION FOR SEQ ID NO: 1:
   (i) SEQUENCE CHARACTERISTICS:
      (A) LENGTH: 5177 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION:1071..2270
   (xi) SEQUENCE DESCRIPTION: SEQ ID NO: 1:
   TTTGAAAGAC CCCACCCGTA GGTGGCAAGC TAGCTTAAGT AACGCCACTT TGCAAGGCAT 60
   GGAAAAATAC ATAACTGAGA ATAGAAAAGT TCAGATCAAG GTCAGGAACA AAGAAACAGC 120
   TGAATACCAA ACAGGATATC TGTGGTAAGC GGTTCCTGCC CCGGCTCAGG GCCAAGAACA 180
   GATGAGACAG CTGAGTGATG GGCCAAACAG GATATCTGTG GTAAGCAGTT CCTGCCCCGG 240
   CTCGGGGCCA AGAACAGATG GTCCCCAGAT GCGGTCCAGC CCTCAGCAGT TTCTAGTGAA 300
   TCATCAGATG TTTCCAGGGT GCCCCAAGGA CCTGAAAATG ACCCTGTACC TTATTTGAAC 360
   TAACCAATCA GTTCGCTTCT CGCTTCTGTT CGCGCGCTTC CGCTCTCCGA GCTCAATAAA 420
   AGAGCCCACA ACCCCTCACT CGGCGCGCCA GTCTTCCGAT AGACTGCGTC GCCCGGGTAC 480
   CCGTATTCCC AATAAAGCCT CTTGCTGTTT GCATCCGAAT CGTGGTCTCG CTGTTCCTTG 540
   GGAGGGTCTC CTCTGAGTGA TTGACTACCC ACGACGGGGG TCTTTCATTT GGGGGCTCGT 600
   CCGGGATTTG GAGACCCCTG CCCAGGGACC ACCGACCCAC CACCGGGAGG TAAGCTGGCC 660
   AGCAACTTAT CTGTGTCTGT CCGATTGTCT AGTGTCTATG TTTGATGTTA TGCGCCTGCG 720
   TCTGTACTAG TTAGCTAACT AGCTCTGTAT CTGGCGGACC CGTGGTGGAA CTGACGAGTT 780
   CTGAACACCC GGCCGCAACC CTGGGAGACG TCCCAGGGAC TTTGGGGGCC GTTTTTGTGG 840
   CCCGACCTGA GGAAGGGAGT CGATGTGGAA TCCGACCCCG TCAGGATATG TGGTTCTGGT 900
   AGGAGACGAG AACCTAAAAC AGTTCCCGCC TCCGTCTGAA TTTTTGCTTT CGGTTTGGAA 960
   CCGAAGCCGC GCGTCTTGTC TGCTGCAGCC AAGCTTGGGC TGCAGGTCGA CTCTAGAGGA 1020
   TCAATTCGGC ACGAGTAAAT CGGTGCTGCC GTCTTTAGGA CATATGAAGT ATG GCA      1076
                                                          Met Ala
                                                            1
   CAG TGG GAT GAC TTT CCT GAT CAG CAA GAG GAC ACT GAC AGC TGT ACA    1124
   Gln Trp Asp Asp Phe Pro Asp Gln Gln Glu Asp Thr Asp Ser Cys Thr
               5                  10                  15
   GAG TCT GTG AAG TTC GAT GCT CGC TCA GTG ACA GCT TTG CTT CCT CCC    1172
   Glu Ser Val Lys Phe Asp Ala Arg Ser Val Thr Ala Leu Leu Pro Pro
        20                  25                  30
   CAT CCT AAA AAT GGC CCA ACT CTT CAA GAG AGG ATG AAG TCT TAT AAA    1220
   His Pro Lys Asn Gly Pro Thr Leu Gln Glu Arg Met Lys Ser Tyr Lys
    35                  40                  45                  50
   ACT GCA CTG ATC ACC CTT TAT CTC ATT GTG TTT GTA GTT CTC GTG CCC    1268
   Thr Ala Leu Ile Thr Leu Tyr Leu Ile Val Phe Val Val Leu Val Pro
                    55                  60                  65
   ATC ATT GGC ATA GTG GCA GCT CAG CTC CTG AAA TGG GAA ACG AAG AAT    1316
   Ile Ile Gly Ile Val Ala Ala Gln Leu Leu Lys Trp Glu Thr Lys Asn
                70                  75                  80
   TGC ACG GTT GGC TCA GTT AAT GCA GAT ATA TCT CCA AGT CCG GAA GGC    1364
   Cys Thr Val Gly Ser Val Asn Ala Asp Ile Ser Pro Ser Pro Glu Gly
            85                  90                  95
   AAA GGA AAT GGC AGT GAA GAT GAA ATG AGA TTT CGA GAA GCT GTG ATG    1412
   Lys Gly Asn Gly Ser Glu Asp Glu Met Arg Phe Arg Glu Ala Val Met
       100                 105                 110
   GAA CGC ATG AGC AAC ATG GAA AGC AGA ATC CAG TAT CTT TCA GAT AAT    1460
   Glu Arg Met Ser Asn Met Glu Ser Arg Ile Gln Tyr Leu Ser Asp Asn
   115                 120                 125                 130
   GAA GCC AAT CTC CTA GAT GCT AAG AAT TTC CAA AAT TTC AGC ATA ACA 1508
   Glu Ala Asn Leu Leu Asp Ala Lys Asn Phe Gln Asn Phe Ser Ile Thr
   135 140 145
   ACT GAT CAA AGA TTT AAT GAT GTT CTT TTC CAG CTA AAT TCC TTA CTT 1556
   Thr Asp Gln Arg Phe Asn Asp Val Leu Phe Gln Leu Asn Ser Leu Leu
   150 155 160
   TCC TCC ATC CAG GAA CAT GAG AAT ATC ATA GGG GAT ATC TCC AAG TCA 1604
   Ser Ser Ile Gln Glu His Glu Asn Ile Ile Gly Asp Ile Ser Lys Ser
   165 170 175
   TTA GTA GGT CTG AAC ACC ACA GTA CTT GAT TTG CAG TTC AGT ATT GAA 1652
   Leu Val Gly Leu Asn Thr Thr Val Leu Asp Leu Gln Phe Ser Ile Glu
   180 185 190
   ACA CTG AAT GGC AGA GTC CAA GAG AAT GCA TTT AAA CAA CAA GAG GAG 1700
   Thr Leu Asn Gly Arg Val Gln Glu Asn Ala Phe Lys Gln Gln Glu Glu
   195 200 205 210
   ATG CGT AAA TTA GAG GAG CGT ATA TAC AAT GCA TCA GCA GAA ATT AAG 1748
   Met Arg Lys Leu Glu Glu Arg Ile Tyr Asn Ala Ser Ala Glu Ile Lys
   215 220 225
   TCT CTA GAT GAA AAA CAA GTA TAT TTG GAA CAG GAA ATA AAA GGG GAA 1796
   Ser Leu Asp Glu Lys Gln Val Tyr Leu Glu Gln Glu Ile Lys Gly Glu
   230 235 240
   ATG AAA CTG TTG AAT AAT ATC ACT AAT GAT CTG AGG CTG AAG GAT TGG 1844
   Met Lys Leu Leu Asn Asn Ile Thr Asn Asp Leu Arg Leu Lys Asp Trp
   245 250 255
   GAA CAT TCT CAG ACA TTG AAA AAT ATC ACT TTA CTC CAA GGT GCC AGA 1892
   Glu His Ser Gln Thr Leu Lys Asn Ile Thr Leu Leu Gln Gly Ala Arg
   260 265 270
   AAG TGC TCG CTG ACT GGG AAA TGG ACC AAC GAT CTG GGC TCC AAC ATG     1940
   Lys Cys Ser Leu Thr Gly Lys Trp Thr Asn Asp Leu Gly Ser Asn Met
   275                 280                 285                 290
   ACC ATC GGG GCT GTG AAC AGC AGA GGT GAA TTC ACA GGC ACC TAC ATC     1988
   Thr Ile Gly Ala Val Asn Ser Arg Gly Glu Phe Thr Gly Thr Tyr Ile
                   295                 300                 305
   ACA GCC GTA ACA GCC ACA TCA AAT GAG ATC AAA GAG TCA CCA CTG CAT     2036
   Thr Ala Val Thr Ala Thr Ser Asn Glu Ile Lys Glu Ser Pro Leu His
               310                 315                 320
   GGG ACA CAA AAC ACC ATC AAC AAG AGG ACC CAG CCC ACC TTT GGC TTC     2084
   Gly Thr Gln Asn Thr Ile Asn Lys Arg Thr Gln Pro Thr Phe Gly Phe
           325                 330                 335
   ACC GTC AAT TGG AAG TTT TCA GAG TCC ACC ACT GTC TTC ACG GGC CAG     2132
   Thr Val Asn Trp Lys Phe Ser Glu Ser Thr Thr Val Phe Thr Gly Gln
       340                 345                 350
   TGC TTC ATA GAC AGG AAT GGG AAG GAG GTC CTG AAG ACC ATG TGG CTG     2180
   Cys Phe Ile Asp Arg Asn Gly Lys Glu Val Leu Lys Thr Met Trp Leu
   355                 360                 365                 370
   CTG CGG TCA AGT GTT AAT GAC ATT GGT GAT GAC TGG AAA GCT ACC AGG     2228
   Leu Arg Ser Ser Val Asn Asp Ile Gly Asp Asp Trp Lys Ala Thr Arg
                   375                 380                 385
   GTC GGC ATC AAC ATC TTC ACT CGC CTG CGC ACA CAG AAG GAG             2270
   Val Gly Ile Asn Ile Phe Thr Arg Leu Arg Thr Gln Lys Glu
               390                 395                 400
   TGAGTGAGTG ACCAAGGTCC TCCTGGACTC CAGGTGAAAA AGGAGATAGA GGCCCTCCTG    2330
   GACAAAATGG TATACCAGGC TTTCCAGGTC TAATAGGTAC TCCAGGTCTT AAAGGTGATC    2390
   GGGGGGATCT CTGGTTTACC TGGAGTTCGA GGATTCCCAG GACCAATGGG GAAGACCGGG    2450
   AAGCCAGGAC TTAATGGACA AAAAGGCCAG AAGGGAGAAA AAGGGAGTGG AAGCATGCAA 2510
   AGACAATCTA ATACAGTCCG ACTGGTGGGT GGCAGCGGCC CTCACGAAGG CAGAGTGGAG 2570
   ATTTTTCACG AAGGCCAGTG GGGTACGGTG TGTGACGACC GCTGGGAACT GCGTGGAGGA 2630
   CTGGTCGTCT GCAGGAGCTT GGGATACAAA GGTGTTCAAA GTGTGCATAA GCGAGCTTAT 2690
   TTTGGAAAAG GTACGGGTCC AATATGGCTG AATGAAGTAT TTTGTTTCGG GAAAGAGTCA 2750
   TCCATTGAAG AGTGCAGAAT TAGACAGTGG GGTGTGAGAG CCTGTTCGCA CGACGAAGAT 2810
   GCTGGGGGTC ACTTTGCACC TACATAATGC ATCATATTTT CATTCACATT TTTTAAACTG 2870
   TTATAAAGTG ATTTTTTTCC TTTGCTTCAC TAAAATCAGC TTAATTAATA TTTAAGAAAC 2930
   TAAGAATTTT ATCCACAGAA AAGGAATATT TAAAAATCAC TGGATAAACA TATAAAATAG 2990
   CTTCATATTT GCTTCAAATA CCAGAACCAT TTCAACTTCT CTAGGTTTTT AAGTGGCTCG 3050
   TGCCGAATTG ATCCCCTCAG GATATAGTAG TTTCGCTTTT GCATAGGGAG GGGGAAATGT 3110
   AGTCTTATGC AATACTCTTG TAGTCTTGCA ACATGGTAAC GATGAGTTAG CAACATGCCT 3170
   TACAAGGAGA GAAAAAGCAC CGTGCATGCC GATTGGTGGA AGTAAGGTGG TACGATCGTG 3230
   CCTTATTAGG AAGGCAACAG ACGGGTCTGA CATGGATTGG ACGAACCACT GAATTCCGCA 3290
   TTGCAGAGAT ATTGTATTTA AGTGCCTAGC TCGATACAGC AAACGCCATT TGACCATTCA 3350
   CCACATTGGT GTGCACCTCC AAGCTTCACG CTGCCGCAAG CACTCAGGGC GCAAGGGCTG 3410
   CTAAAGGAAG CGGAACACGT AGAAAGCCAG TCCGCAGAAA CGGTGCTGAC CCCGGATGAA 3470
   TGTCAGCTAC TGGGCTATCT GGACAAGGGA AAACGCAAGC GCAAAGAGAA AGCAGGTAGC 3530
   TTGCAGTGGG CTTACATGGC GATAGCTAGA CTGGGCGGTT TTATGGACAG CAAGCGAACC 3590
   GGAATTGCCA GCTGGGGCGC CCTCTGGTAA GGTTGGGAAG CCCTGCAAAG TAAACTGGAT 3650
   GGCTTTCTTG CCGCCAAGGA TCTGATGGCG CAGGGGATCA AGATCTGATC AAGAGACAGG 3710
   ATGAGGATCG TTTCGCATGA TTGAACAAGA TGGATTGCAC GCAGGTTCTC CGGCCGCTTG 3770
   GGTGGAGAGG CTATTCGGCT ATGACTGGGC ACAACAGACA ATCGGCTGCT CTGATGCCGC 3830
   CGTGTTCCGG CTGTCAGCGC AGGGGCGCCC GGTTCTTTTT GTCAAGACCG ACCTGTCCGG 3890
   TGCCCTGAAT GAACTGCAGG ACGAGGCAGC GCGGCTATCG TGGCTGGCCA CGACGGGCGT 3950
   TCCTTGCGCA GCTGTGCTCG ACGTTGTCAC TGAAGCGGGA AGGGACTGGC TGCTATTGGG 4010
   CGAAGTGCCG GGGCAGGATC TCCTGTCATC TCACCTTGCT CCTGCCGAGA AAGTATCCAT 4070
   CATGGCTGAT GCAATGCGGC GGCTGCATAC GCTTGATCCG GCTACCTGCC CATTCGACCA 4130
   CCAAGCGAAA CATCGCATCG AGCGAGCACG TACTCGGATG GAAGCCGGTC TTGTCGATCA 4190
   GGATGATCTG GACGAAGAGC ATCAGGGGCT CGCGCCAGCC GAACTGTTCG CCAGGCTCAA 4250
   GGCGCGCATG CCCGACGGCG AGGATCTCGT CGTGACCCAT GGCGATGCCT GCTTGCCGAA 4310
   TATCATGGTG GAAAATGGCC GCTTTTCTGG ATTCATCGAC TGTGGCCGGC TGGGTGTGGC 4370
   GGACCGCTAT CAGGACATAG CGTTGGCTAC CCGTGATATT GCTGAAGAGC TTGGCGGCGA 4430
   ATGGGCTGAC CGCTTCCTCG TGCTTTACGG TATCGCCGCT CCCGATTCGC AGCGCATCGC 4490
   CTTCTATCGC CTTCTTGACG AGTTCTTCTG AGCGGGACTC TGGGGTTCGA TAAAATAAAA 4550
   GATTTTATTT AGTCTCCAGA AAAAGGGGGG AATGAAAGAC CCCACCTGTA GGTTTGGCAA 4610
   GCTAGCTTAA GTAACGCCAT TTTGCAAGGC ATGGAAAAAT ACATAACTGA GAATAGAGAA 4670
   GTTCAGATCA AGGTCAGGAA CAGATGGAAC AGCTGAATAT GGGCCAAACA GGATATCTGT 4730
   GGTAAGCAGT TCCTGCCCCG GCTCAGGGCC AAGAACAGAT GGAACAGCTG AATATGGGCC 4790
   AAACAGGATA TCTGTGGTAA GCAGTTCCTG CCCCGGCTCA GGGCCAAGAA CAGATGGTCC 4850
   CCAGATGCGG TCCAGCCCTC AGCAGTTTCT AGAGAACCAT CAGATGTTTC CAGGGTGCCC 4910
   CAAGGACCTG AAATGACCCT GTGCCTTATT TGAACTAACC AATCAGTTCG CTTCTCGCTT 4970
   CTGTTCGCGC GCTTCTGCTC CCCGAGCTCA ATAAAAGAGC CCACAACCCC TCACTCGGGG 5030
   CGCCAGTCCT CCGATTGACT GAGTCGCCCG GGTACCCGTG TATCCAATAA ACCCTCTTGC 5090
   AGTTGCATCC GACTTGTGGT CTCGCTGTTC CTTGGGAGGG TCTCCTCTGA GTGATTGACT 5150
   ACCCGTCAGC GGGGGTCTTT CATTTGG 5177
(2) INFORMATION FOR SEQ ID NO: 2:
   (i) SEQUENCE CHARACTERISTICS:
      (A) LENGTH: 400 amino acids
      (B) TYPE: amino acid
      (D) TOPOLOGY: linear
   (ii) MOLECULE TYPE: protein
   (xi) SEQUENCE DESCRIPTION: SEQ ID NO: 2:
   Met Ala Gln Trp Asp Asp Phe Pro Asp Gln Gln Glu Asp Thr Asp Ser
   1 5 10 15
   Cys Thr Glu Ser Val Lys Phe Asp Ala Arg Ser Val Thr Ala Leu Leu
   20 25 30
   Pro Pro His Pro Lys Asn Gly Pro Thr Leu Gln Glu Arg Met Lys Ser
   35 40 45
   Tyr Lys Thr Ala Leu Ile Thr Leu Tyr Leu Ile Val Phe Val Val Leu
   50 55 60
   Val Pro Ile Ile Gly Ile Val Ala Ala Gln Leu Leu Lys Trp Glu Thr
   65 70 75 80
   Lys Asn Cys Thr Val Gly Ser Val Asn Ala Asp Ile Ser Pro Ser Pro
   85 90 95
   Glu Gly Lys Gly Asn Gly Ser Glu Asp Glu Met Arg Phe Arg Glu Ala
   100 105 110
   Val Met Glu Arg Met Ser Asn Met Glu Ser Arg Ile Gln Tyr Leu Ser
   115 120 125
   Asp Asn Glu Ala Asn Leu Leu Asp Ala Lys Asn Phe Gln Asn Phe Ser
   130 135 140
   Ile Thr Thr Asp Gln Arg Phe Asn Asp Val Leu Phe Gln Leu Asn Ser
   145 150 155 160
   Leu Leu Ser Ser Ile Gln Glu His Glu Asn Ile Ile Gly Asp Ile Ser
   165 170 175
   Lys Ser Leu Val Gly Leu Asn Thr Thr Val Leu Asp Leu Gln Phe Ser
   180 185 190
   Ile Glu Thr Leu Asn Gly Arg Val Gln Glu Asn Ala Phe Lys Gln Gln
   195 200 205
   Glu Glu Met Arg Lys Leu Glu Glu Arg Ile Tyr Asn Ala Ser Ala Glu
   210 215 220
   Ile Lys Ser Leu Asp Glu Lys Gln Val Tyr Leu Glu Gln Glu Ile Lys
   225 230 235 240
   Gly Glu Met Lys Leu Leu Asn Asn Ile Thr Asn Asp Leu Arg Leu Lys
   245 250 255
   Asp Trp Glu His Ser Gln Thr Leu Lys Asn Ile Thr Leu Leu Gln Gly
   260 265 270
   Ala Arg Lys Cys Ser Leu Thr Gly Lys Trp Thr Asn Asp Leu Gly Ser
   275 280 285
   Asn Met Thr Ile Gly Ala Val Asn Ser Arg Gly Glu Phe Thr Gly Thr
   290 295 300
   Tyr Ile Thr Ala Val Thr Ala Thr Ser Asn Glu Ile Lys Glu Ser Pro
   305 310 315 320
   Leu His Gly Thr Gln Asn Thr Ile Asn Lys Arg Thr Gln Pro Thr Phe
   325 330 335
   Gly Phe Thr Val Asn Trp Lys Phe Ser Glu Ser Thr Thr Val Phe Thr
   340 345 350
   Gly Gln Cys Phe Ile Asp Arg Asn Gly Lys Glu Val Leu Lys Thr Met
   355 360 365
   Trp Leu Leu Arg Ser Ser Val Asn Asp Ile Gly Asp Asp Trp Lys Ala
   370 375 380
   Thr Arg Val Gly Ile Asn Ile Phe Thr Arg Leu Arg Thr Gln Lys Glu
   385 390 395 400